# FASCICULE DE BREVET EUROPEEN

(11) **EP 0 427 587 B1**
(45) Date de publication et mention de la délivrance du brevet: **15.01.1997**
(21) Numéro de dépôt: 90403010.3
(22) Date de dépôt: 25.10.1990
(51) Int. Cl.: C07H 19/073

(54) **Procédé de préparation de l'AZT (azido-3'-désoxy-3'-thymidine) et de composés apparentés**
Verfahren zur Herstellung von AZT(3'-azido-3'-desoxythymidin) und diebezüglichen Verbindungen
Process for the preparation of AZT (3'-azido-3'-deoxy-thymidine) and related compounds

(30) Priorité: 27.10.1989 FR 8914132
(43) Date de publication de la demande: 15.05.1991
(73) Titulaire: UNIVERSITE PIERRE ET MARIE CURIE PARIS VI, 75230 Paris Cedex 05 (FR)
(72) Inventeur: Czernecki, Stanislas, F-77590 Maincy (FR); Valéry, Jean-Marc, F-77176 Nandy (FR)
(74) Mandataire: Warcoin, Jacques

(56) Documents cités:
- EP-A- 0 199 451
- EP-A- 0 325 537
- WO-A-88/05657
- US-A- 4 681 933
- JOURNAL OF MEDICINAL CHEMISTRY, vol. 26, 1983, American Chemical Society; T.-S. LIN et al., pp. 544-548
- EUROPEAN JOURNAL OF MEDICAL CHEMISTRY - CHIM. THER., vol. 20, no. 4, 1985; L. COLLA et al., pp. 295-301
- JOURNAL OF ORGANIC CHEMISTRY, vol. 38, no. 25, 1973; R.P. GLINSKI et al., pp. 4299-4305
- CHEMICAL ABSTRACTS, vol. 88, 1978, Columbus, OH (US); pp. 590-591, AN 12166x
- BULLETIN OF THE CHEMICAL SOCIETY OF JAPAN, vol. 52, no. 4, avril 1979; J. KIMURA et al., pp. 1191-1196
- JUSTUS LIEBIGS ANNALEN DER CHEMIE, 1978, Verlag Chemie GmbH, Weinheim (DE); H. LOIBNER et al., pp. 78-86

## Description

La présente invention concerne un procédé de synthèse de la (azido-3-didésoxy-2,3, β -D-érythropentofurannosyl)-1-thymine dénommé également azido-3'-désoxy-3'-thymidine ou AZT et de composés apparentés présentant des propriétés antivirales et utilisés notamment dans la lutte contre le SIDA.

Différentes synthèses de l'AZT ont été décrites. Les plus directes sont réalisées à partir de la thymidine mais comportent un assez grand nombre d'étapes et donnent, dans la majorité des cas, des rendements faibles.

Cependant, deux procédés de synthèse ont été décrits, qui ne comportent que trois étapes.

Ainsi, l'article publié en 1984 (V.E. ZAITSEVA et Coll., Bioorg. Khim., 10, 670 (1984)) décrit une synthèse de ce type mais qui semble-t-il, n'a pas été développée, compte tenu du fait sans doute que la seconde étape du procédé nécessite la préparation de p-méthylbenzoate de lithium, et que l'isolement de l'AZT obtenu dans la dernière étape se fait par chromatographie sur colonne.

Un brevet allemand DE 37 05 794 A1, de Y. HAYAUCHI et O. LOCKHOFF, publié en 1988, décrit également ce type de procédé en trois étapes qui fournit l'AZT à partir de la thymidine, mais avec un rendement de seulement 42,5%.

Ce procédé nécessite par ailleurs l'utilisation de réactifs au silicium et de fluorure de tétraalkylammonium, qui sont tous deux des composés coûteux.

Par ailleurs, le document EP-A-325 537 décrit la préparation d'AZT et de composés apparentés à partir de thymidine dont le radical hydroxyle en 5' est protégé par un groupement acyle, puis réaction subséquente pour obtenir les composés souhaités.

Le document WO-88/05657, notamment dans le schéma de synthèse de la page 15, décrit la préparation d'un composé de type 3'-azido-2',3'-didéoxyuridine par l'intermédiaire d'un composé de type 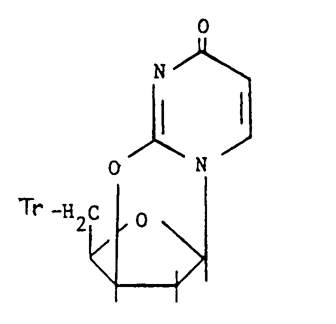 Tr correspondant au radical trityle.

Ce composé intermédiaire est obtenu en deux étapes via un intermédiaire protégé par un groupe mésylate en 3' avant cyclisation avec la soude.

La présente invention a pour objet un procédé de préparation d'AZT et de composés apparentés en trois étapes mais qui présente les avantages de pouvoir être réalisé dans seulement deux milieux réactionnels successifs, de mettre en oeuvre des réactifs de faible coût et de conduire aisément à l'AZT ou à l'analogue souhaité avec un état de pureté satisfaisant.

Plus particulièrement, la présente invention se rapporte à un procédé de préparation d'un composé de formule (I) 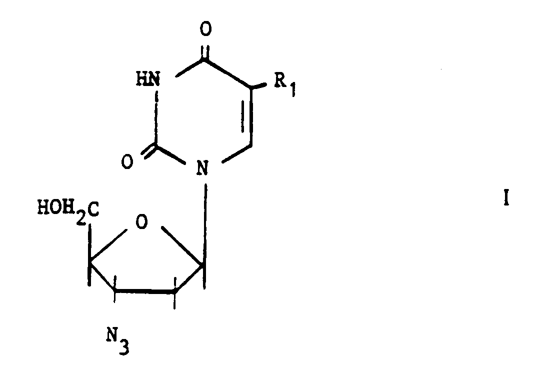 dans laquelle :
R₁ est H, un radical alkyle, un radical alcoxy, hydroxyalkyle ou halogène,
à partir d'un composé de formule (II) 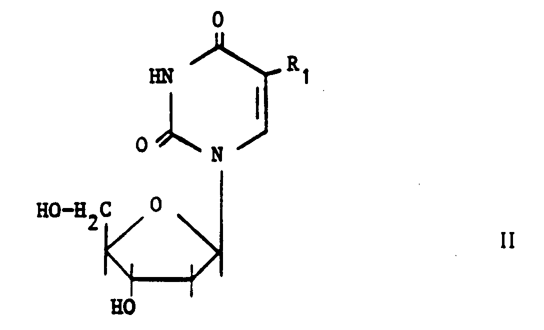 caractérisé en ce que l'on fait réagir le composé de formule II avec un dérivé

R₂X

dans lequel :
X représente un halogène et
R₂ un radical trityle, un radical mono ou dialkoxy en C₁₋₃ trityle, un radical pixyle ou de type (R₃R₄R₅)Si où R₃, R₄ et R₅ indépendamment les uns des autres représentent un groupe alkyle de 1 à 5 atomes de carbone ou un groupe aryle
de façon à obtenir un composé de formule III 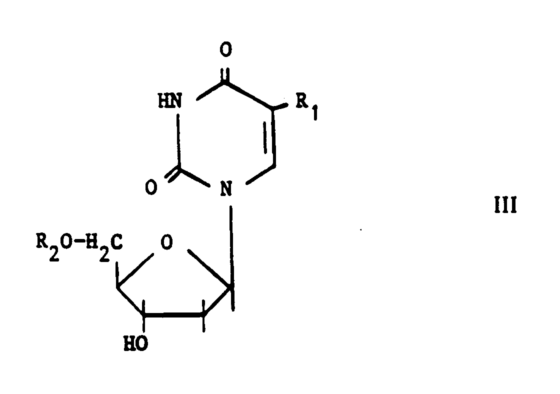 qui est ensuite transformé sous l'action d'un diester d'acide azodicarboxylique et d'un dérivé de phosphine ou phosphite dans un solvant compatible avec les conditions de réaction pour former un composé de formule IV 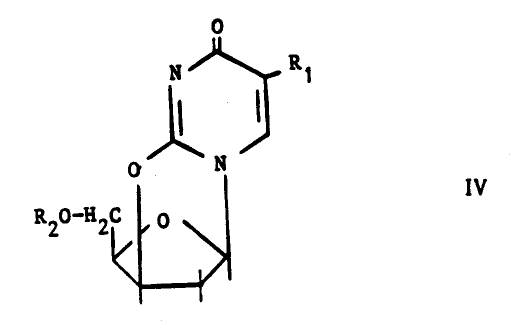 lequel, après une éventuelle séparation, est ouvert en présence d'un azoture métallique dans un solvant compatible avec les conditions de réaction pour conduire à un composé de formule V 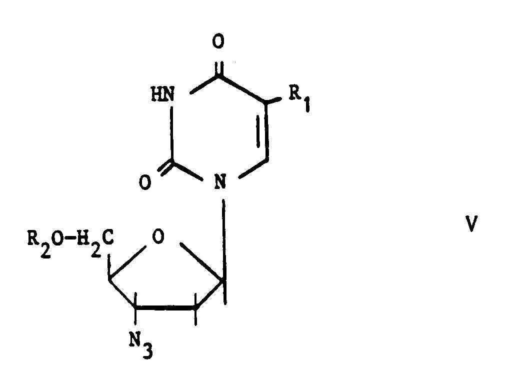 qui, par déprotection de la fonction 5′, donne le composé de formule I.

Bien entendu, parmi les composés de formule (I), il faut citer plus particulièrement l'AZT, c'est-à-dire le composé dans lequel R₁ est égal à CH₃ ; néanmoins, actuellement, d'autres dérivés azido-3′ sont développés, en particulier ceux où R₁ est égal à un radical alkyle inférieur, un radical alcoxy inférieur ou hydroxyalkyle inférieur, comportant de 1 à 5 atomes de carbone, par exemple des radicaux méthyle ou éthyle.

Parmi ces autres dérivés, on peut également citer l'azido-3′-désoxy-3′-uridine.

Dans le procédé selon la présente invention, le produit de départ, en particulier pour préparer l'AZT, est la thymidine, composé qui est disponible industriellement et qui doit, pour les besoins du procédé, être désséché car la présence d'eau nuit considérablement au rendement du procédé.

Parmi les réactifs du type R₂X, on choisira de préférence le chlorure de trityle, composé de faible coût et facilement disponible.

Une autre caractéristique importante du procédé réside dans le fait qu'il est inutile de purifier ou d'isoler le composé (III) ainsi obtenu à la condition que l'on utilise des conditions compatibles avec la préparation du composé anhydro-2,3′ de formule (IV).

De telles conditions consistent, par exemple, à associer un solvant aprotique anhydre et une base organique ou minérale de force suffisante.

Parmi les solvants, il faut citer le DMF, le HMPT, le DMSO. Pour des raisons de coût, le DMF est le solvant préféré, compte tenu du fait qu'il permet à la fois la préparation directe du composé de formule IV, mais aussi la mise en oeuvre de l'ouverture de celui-ci sans avoir à l'isoler.

Parmi les bases organiques, il faut citer les amines tertiaires telles que la diméthylamino-4-pyridine (DMAP), le diaza-1,4-bicyclo[4.3.0] nonène-5 (DBN), le diaza-1,4-bicyclo[2.2.2]octane (DABCO), le diaza-1,8-bicyclo[5.4.0]undécène-7 (DBU), la triéthyl- ou tri-n-butylamine.

Parmi les bases minérales, les carbonates alcalins sont convenables.

L'obtention du composé de formule (IV) résulte de l'action, dans des conditions telles que celles qui sont définies ci-dessus, d'un diester de l'acide azodicarboxylique R₆O-OC-N = N-CO-OR₆ en présence d'un dérivé de phosphine ou de phosphite.

Parmi ceux-ci, la phosphine préférée est la triphénylphosphine qui est facilement accessible industriellement.

De même, les diesters de l'acide azodicarboxylique peuvent être des esters d'aryle ou d'alkyle inférieur (R₇ est égal à un groupe alkyle comportant de 1 à 4 atomes de carbone). Mais les produits préférés seront les azodicarboxylates de diisopropyle (DIAD) ou de diéthyle (DEAD), car ils sont tous deux facilement accessibles.

Ces différents réactifs sont utilisés, de préférence, en excès molaire par rapport au composé de formule (III).

Pour cette réaction, les conditions de température et de pression ne sont pas, à proprement parler caractéristiques. En effet, la réaction est complète au bout de quelques heures à la température ordinaire. Mais on peut la prolonger davantage sans aucun inconvénient.

A l'issue de ce stade du procédé, on obtient le composé de formule (IV) qui peut être soit traité sans aucune séparation préalable comme cela sera décrit ci-après, soit séparé du mélange réactionnel.

Dans ce cas, la séparation se fait par simple filtration, soit après ajoût d'un tiers solvant tel que l'éther diéthylique, l'éther diisopropylique ou l'acétate de n-butyle qui provoque la précipitation totale du composé de formule (IV), soit en utilisant un faible volume de solvant et/ou une basse température lors de la séparation.

Le composé de formule (IV) ainsi obtenu a l'avantage de présenter une bonne solubilité, ce qui facilite la mise en oeuvre de l'étape suivante.

La réaction suivante qui est la réaction d'ouverture est effectuée grâce à un excès d'azoture dans un solvant polaire. Cet excès peut aller de 1,5 à 15 équivalents par mole de composé de formule (IV). Les azotures alcalins peuvent être utilisés. Il est possible, dans certains cas, de limiter l'excès de réactif en faisant la réaction en présence d'un autre sel de lithium.

Le solvant utilisé est, en général, un solvant aprotique polaire compatible avec la réaction, c'est-à-dire permettant de solubiliser aussi bien le composé de formule (IV) que l'azoture utilisé.

C'est pourquoi cette réaction sera effectuée, par exemple dans le DMF, ou dans un solvant homologue supérieur, à chaud, c'est-à-dire à des températures de l'ordre de 100°C jusqu'à la température de reflux, qui devront rester compatibles avec la stabilité des produits mis en jeu.

La réaction sera alors complète en quelques heures, à savoir environ 7 à 10 heures.

La séparation du composé de formule (V) formé à la fin de cette étape peut être effectuée en traitant le mélange réactionnel avec un hydrogénocarbonate alcalin puis en extrayant avec un solvant halogéné tel que le chloroforme.

La phase organique est alors lavée à l'eau, séchée et évaporée afin d'obtenir un produit homogène. Ce composé est alors déprotégé puis extrait.

Il est bien entendu possible, directement à partir du milieu réactionnel conduisant au composé de formule (V), de déprotéger celui-ci.

Cette déprotection est effectuée à l'aide d'un réactif acide de type acide de Lewis, tel que l'éthérate de trifluorure de bore, ou protique comme par exemple les hydracides halogénés et/ou l'acide acétique en présence d'eau.

L'élimination des différents sous-produits organiques se fait simplement en traitant le mélange réactionnel brut par une base alcaline aqueuse, puis en extrayant à l'éther.

La neutralisation de la phase aqueuse résultante au moyen d'un acide convenable permet ensuite de récupérer le composé de formule (I), en particulier l'AZT, de façon quasi quantitative.

Les exemples donnés ci-après, à titre non limitatif, permettront de mettre en évidence d'autres avantages et caractéristiques de la présente invention.

### EXEMPLE 1

4,95g de thymidine et 6,83g de chlorure de trityle dans 60 ml de pyridine sont chauffés à 110°C pendant 1 heure et 30 minutes. La chromatographie sur couche mince ou C.C.M. (éluant : acétate d'éthyle) montre que la réaction est terminée.

Le mélange réactionnel est alors versé lentement dans 200g de glace et 200 ml d'eau sous vive agitation. Après 30 minutes, le précipité blanc formé est essoré, lavé à l'eau froide (50ml) et séché. On obtient 7,4g de III (75,5%).
Tf = 128-130°C

| Analyse élémentaire | | | |
|---|---|---|---|
| | C | H | N |
| Calculé | 71,88% | 5,82% | 5,78% |
| Trouvé | 71,83% | 5,85% | 5,71% |

### EXEMPLE 2

4,85g de III et 3,93g de triphénylphosphine sont dissous dans 50ml de DMF. On ajoute goutte à goutte 2,95ml de DIAD. La réaction est légèrement exothermique et peut être refroidie par un bain d'eau.

Après 30 minutes, la C.C.M. (éluants : acétate d'éthyle et chloroforme-méthanol 6-1) montre qu'il ne reste plus de produit de départ. Le milieu réactionnel est alors versé dans 500ml d'éther et agité 1 heure à 0°C. Le précipité obtenu est essoré, lavé trois fois par 20ml d'éther et séché.

On obtient 3,78g de IV (81%).
Tf = 152°C.
Les analyses RMN et IR confirment la structure du composé IV.

| Analyse élémentaire | | | |
|---|---|---|---|
| | C | H | N |
| Calculé | 74,66% | 5,62% | 6% |
| Trouvé | 74,66% | 5,59% | 5,95% |

### EXEMPLE 3

A une solution de 242mg de thymidine dans 3ml de DMF, on ajoute successivement 335mg de chlorure de trityle et 127mg de carbonate de sodium.

Après 5 heures à 110°C, on laisse le mélange réactionnel revenir à température ordinaire et on ajoute 393mg de triphénylphosphine. Apr̂ès dissolution, on introduit 0,3 ml de DIAD.

La réaction est terminée en quelques heures et le mélange réactionnel est traité comme dans l'Exemple 2.

On obtient 300mg de IV (64%).

### EXEMPLE 4

940mg de IV et 265mg d'azoture de sodium dans 5ml de DMF sont chauffés à 140°C pendant 6 heures.

Le mélange réactionnel est versé dans une solution saturée d'hydrogénocarbonate de sodium (20ml) et extrait au chloroforme (3x15ml).

Après lavage à l'eau, séchage et évaporation du solvant, on obtient 1,08g de V, homogène en C.C.M. (éluant : acétate d'éthyle).

Le spectre RMN montre la présence d'environ 5% de DMF.

La réaction est donc quantitative.

### EXEMPLE 5

4,6g de IV et 1,3g d'azoture de sodium dans 25ml de DMF sont chauffés à 140°C pendant 7 heures.

Un traitement analogue à celui de l'Exemple 4 fournit un sirop qui est dissout dans 30ml d'acide acétique à 80%. Après 15 minutes de chauffage à 120°C, la chromatographie sur couche mince ou C.C.M. montre que la déprotection est terminée.

Le mélange est refroidi à 5°C et traité par 145ml de soude 3M (le pH doit être au moins égal à 9) et l'ensemble extrait à l'éther (2x40ml).

La phase aqueuse résultante est neutralisée par de l'acide chlorhydrique environ 5M, puis évaporée.

La masse cristalline obtenue est reprise par 120ml d'acétate d'éthyle chaud. Les insolubles sont filtrés à chaud et lavés par 20ml d'acétate d'éthyle bouillant.

Après refroidissement, le filtrat est dilué par un volume équivalent de méthanol et traité par du charbon actif. Une filtration sur célite suivie de l'évaporation des solvants fournit 2,45g du composé I (93%) homogène en C.C.M. (éluant : acétate d'éthyl-méthanol 20-1) à l'état vitreux.

La cristallisation dans l'eau fournit 1,92g (73% à partir de IV) d'AZT.
Tf - 117-119°C.

## Revendications

1. Procédé de préparation d'un compose de formule (I) 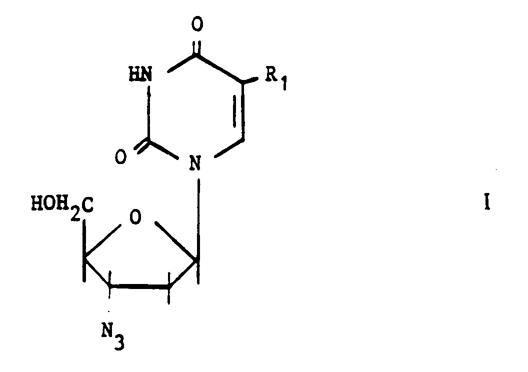 dans laquelle :
R₁ est H, un radical alkyle, un radical alcoxy, hydroxyalkyle ou halogène,
à partir d'un composé de formule (II) 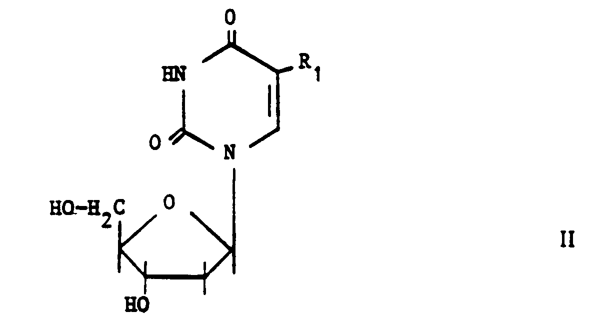 caractérisé en ce que l'on fait réagir le composé de formule II avec un dérivé
R₂X
dans lequel :
X représente un halogène et
R₂ un radical trityle, un radical mono ou dialkoxy en C₁₋₃ trityle, un radical pixyle ou de type (R₃R₄R₅)Si ou R₃, R₄ et R₅ indépendamment les uns des autres représentent un groupe alkyle de 1 à 5 atomes de carbone ou un groupe aryle
de façon à obtenir un composé de formule III 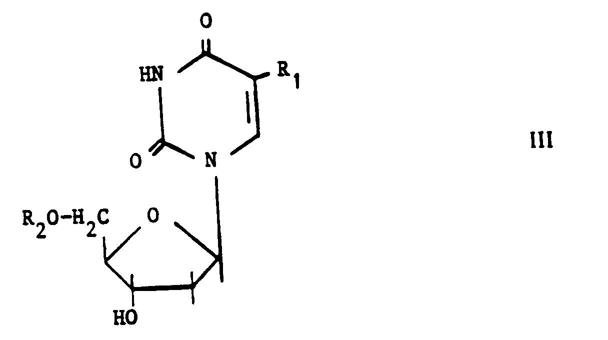 qui est ensuite transformé sous l'action d'un diester d'acide azodicarboxylique et d'un dérivé de phosphine ou phosphite dans un solvant compatible avec les conditions de réaction pour former un composé de formule IV 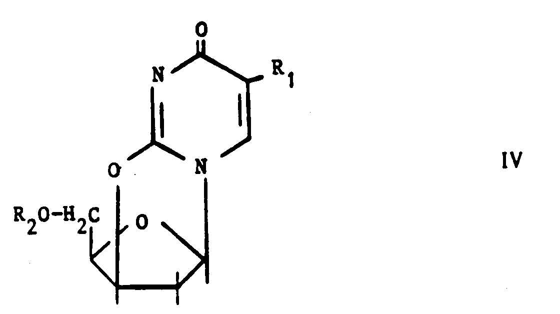 lequel, après une éventuelle séparation, est ouvert en présence d'un azoture métallique dans un solvant compatible avec les conditions de réaction pour conduire à un composé de formule V 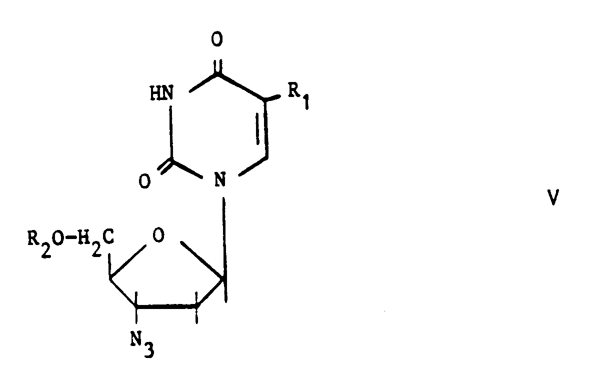 qui, par déprotection de la fonction 5', donne le composé de formule I.

2. Procédé selon la revendication 1, caractérisé en ce que le dérivé R₂X est de préférence le chlorure de trityle.

3. Procédé selon l'une des revendications 1 ou 2, caractérisé en ce qu'il est possible de préparer directement le composé de formule IV à partir du composé de formule III non isolé et ni purifié en utilisant un solvant compatible et des conditions de réaction appropriées aux deux composés.

4. Procédé selon la revendication 3, caractérisé en ce que le solvant est un solvant aprotique anhydre et qu'il lui est associée une base organique ou minérale.

5. Procédé selon la revendication 4, caractérisé en ce que le solvant est choisi parmi le DMF, le HMPT et le DMSO.

6. Procédé selon l'une des revendications 4 ou 5, caractérisé en ce que la base est choisie parmi les amines tertiaires et, de préférence, la diméthylamino-4-pyridine, le diaza-1,4-bicyclo[4.3.0]nonène-5, le diaza-1,4-bicyclo[2.2.2]octane, le diaza-1,8-bicyclo[5.4.0]undécène-7, la triéthylbutylamine, la tri-n-butylamine et les carbonates alcalins.

7. Procédé selon l'une des revendications 1 à 6, caractérisé en ce que la phosphine utilisée est la triphénylphosphine.

8. Procédé selon l'une des revendications 1 à 7, caractérisé en ce que le diester d'acide azodicarboxylique est un ester d'alkyle ou d'aryle.

9. Procédé selon la revendication 8, caractérisé en ce que le diester est de préférence l'azodicarboxylate diisopropyle (DIAD) ou l'azodicarboxylate de diéthyle (DEAD).

10. Procédé selon l'une des revendications 1 à 9, caractérisé en ce que la phosphine ou phosphite et le diester azodicarboxylate sont utilisés en excès molaire par rapport au composé de formule III.

11. Procédé selon l'une des revendications 1 à 10, caractérisé en ce que la réaction d'ouverture est effectuée en présence d'un excès d'azoture alcalin dans un solvant polaire.

12. Procédé selon la revendication 11, caractérisé en ce que cet excès est de l'ordre de 1,5 à 15 équivalents par mole de composé de formule IV.

13. Procédé selon l'une des revendications 11 ou 12, caractérisé en ce que le solvant polaire utilisé est de préférence de DMF.

14. Procédé selon l'une des revendications 1 à 13, caractérisé en ce que la déprotection de la position 5' du composé de formule V est réalisée à l'aide d'un réactif acide du type acide de Lewis ou protique.

15. Procédé selon la revendication 14, caractérisé en ce que cette déprotection est réalisée avec un réactif choisi parmi l'éthérate de trifluorure de bore, les hydracides halogénés et l'acide acétique en présence d'eau.

## Claims

1. Process for preparing a compound of formula (I) 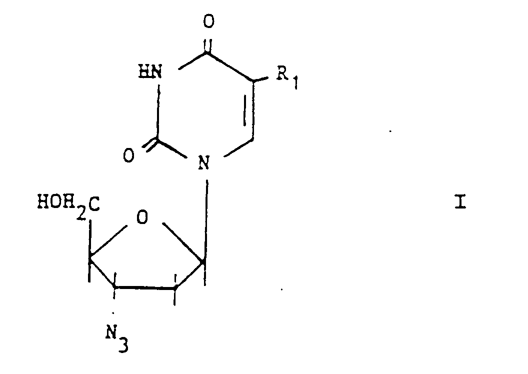 in which:
R₁ is H, an alkyl radical or an alkoxy, hydroxyalkyl or halogen radical,
from a compound of formula (II) 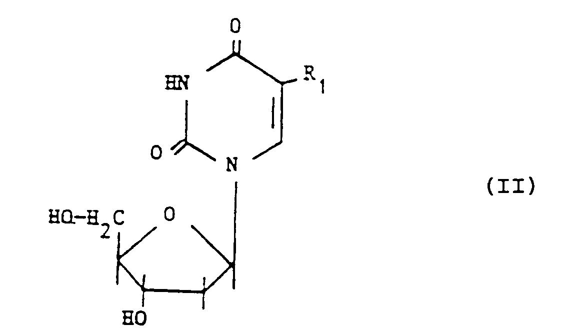 characterized in that the compound of formula II is reacted with a derivative
R₂X
in which:
X represents a halogen, and
R₂ a trityl radical, a (C₁₋₃ mono- or dialkoxy)trityl radical, a pixyl radical or a radical of the type (R₃R₄R₅)Si where R₃, R₄ and R₅, independently of one another, represent an alkyl group having 1 to 5 carbon atoms or an aryl group,
so as to obtain a compound of formula III 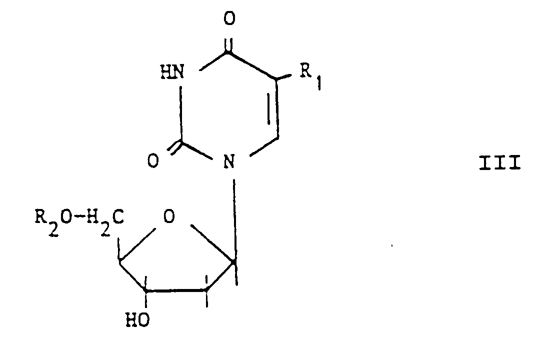 which is then converted, through the action of an azodicarboxylic acid diester and a phosphine or phosphite derivative in a solvent compatible with the reaction conditions, to form a compound of formula IV 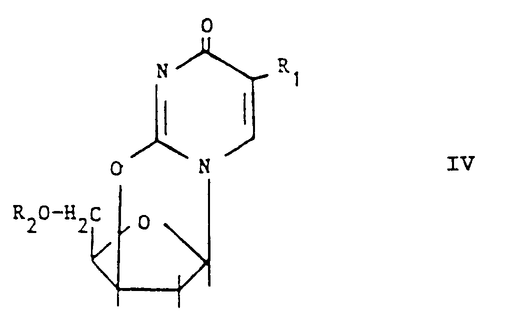 which, after an optional separation, is opened in the presence of a metal azide in a solvent compatible with the reaction conditions to lead to a compound of formula V 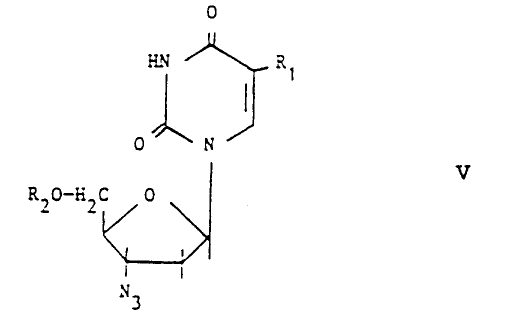 which, on deprotection of the 5' function, gives the compound of formula I.

2. Process according to Claim 1, characterized in that the derivative R₂X is preferably trityl chloride.

3. Process according to one of Claims 1 and 2, characterized in that it is possible to prepare the compound of formula IV directly from the compound of formula III, not isolated or purified, using a compatible solvent and reaction conditions suited to both compouds.

4. Process according to Claim 3, characterized in that the solvent is an anhydrous aprotic solvent and in that an organic or inorganic base is combined therewith.

5. Process according to Claim 4, characterized in that the solvent is selected from DMF, HMPT and DMSO.

6. Process according to one of Claims 4 and 5, characterized in that the base is selected from tertiary amines, and preferably 4-dimethylaminopyridine, 1,4-diazabicyclo[4.3.0]non-5-ene, 1,4-diazobicyclo[2.2.2]octane, 1,8-diazabicyclo[5.4.0]undec-7-ene, triethylbutylamine and tri-n-butylamine, and alkali metal carbonates.

7. Process according to one of Claims 1 to 6, characterized in that the phosphine used is triphenylphosphine.

8. Process according to one of Claims 1 to 7, characterized in that the azodicarboxylic acid diester is an alkyl or aryl ester.

9. Process according to Claim 8, characterized in that the diester is preferably diisopropyl azodicarboxylate (DIAD) or diethyl azodicarboxylate (DEAD).

10. Process according to one of Claims 1 to 9, characterized in that the phosphine or phosphite and the azodicarboxylate diester are used in molar excess relative to the compound of formula III.

11. Process according to one of Claims 1 to 10, characterized in that the opening reaction is performed in the presence of an excess of alkali metal azide in a polar solvent.

12. Process according to Claim 11, characterized in that this excess is of the order of 1.5 to 15 equivalents per mole of compound of formula IV.

13. Process according to one of Claims 11 and 12, characterized in that the polar solvent used is preferably DMF.

14. Process according to one of Claims 1 to 13, characterized in that the deprotection of the 5' position of the compound of formula V is carried out using an acidic reagent of the Lewis acid or protic acid type.

15. Process according to Claim 14, characterized in that this deprotection is carried out with a reagent selected from boron trifluoride etherate, halogen hydracids and acetic acid in the presence of water.

## Patentansprüche

1. Verfahren zur Herstellung einer Verbindung der Formel (I) 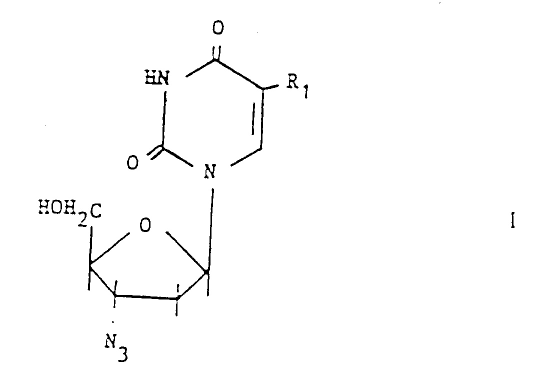 wobei:
R₁ H ein Alkylradikal, ein Alkoxyradikal, Hydroxyalkyl oder Halogen darstellt,
ausgehend von einer Verbindung der Formel (II) 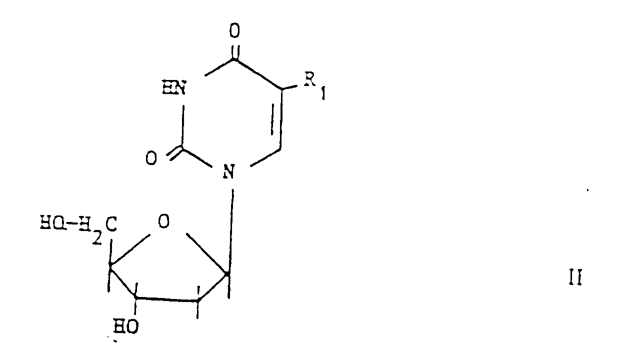 dadurch charakterisiert, daß man eine Verbindung der Formel II mit einem Derivat
R₂X
umsetzt, wobei:
X ein Halogen darstellt und
R₂ ein Tritylradikal, ein Mono- oder Di-C₁-C₃alkoxytritylradikal, ein Pixylradikal oder ein Radikal des Typs (R₃R₄R₅)Si darstellt, wobei R₃, R₄ und R₅ unabhängig voneinander eine Alkylgruppe mit 1 bis 5 Kohlenstoffatomen oder eine Arylgruppe darstellt,
zur Herstellung einer Verbindung der Formel III 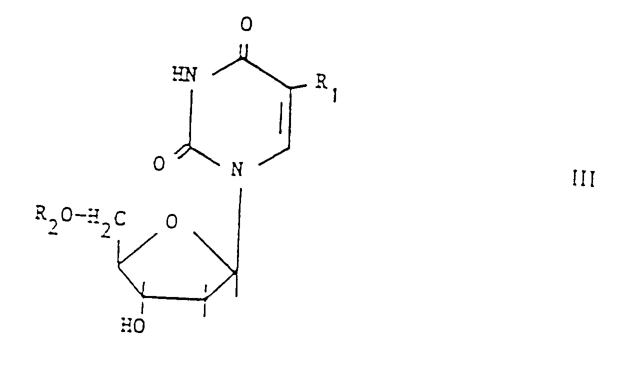 die anschließend durch Einwirkung eines Azodicarbonsäurediesters und eines Phosphinderivats oder Phosphitderivats in einem mit den Reaktionsbedingungen verträglichen Lösungsmittel in eine Verbindung der Formel IV umgewandelt wird 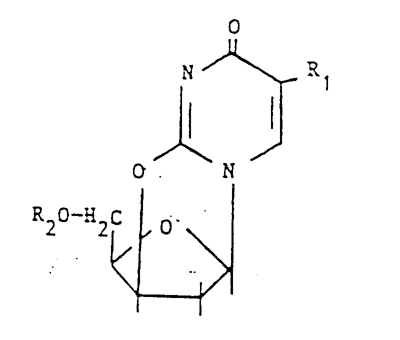 die gegebenenfalls nach Trennung in Gegenwart eines metallischen Azids in einem mit den Reaktionsbedingungen verträglichen Lösungsmittel zur Herstellung einer Verbindung der Formel V geöffnet wird 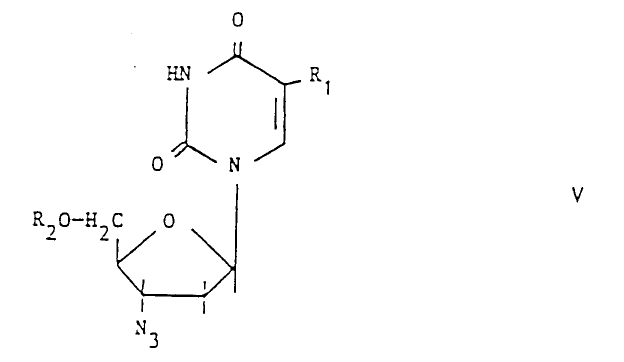 die durch Entschützen der Funktion 5' die Verbindung der Formel I ergibt.

2. Verfahren nach Anspruch 1, dadurch charakterisiert, daß das Derivat R₂X bevorzugt Tritylchlorid ist.

3. Verfahren nach einem der Ansprüche 1 oder 2, dadurch charakterisiert, daß es möglich ist, die Verbindung der Formel IV direkt ausgehend aus der nicht isolierten und nicht gereinigten Verbindung der Formel III herzustellen, indem ein verträgliches Lösungsmittel und Reaktionsbedingungen eingesetzt werden, die für beide Verbindungen geeignet sind.

4. Verfahren nach Anspruch 3, dadurch charakterisiert, daß das Lösungsmittel ein aprotisches wasserfreies Lösungsmittel ist und daß mit ihm eine organische oder mineralische Base assoziiert ist.

5. Verfahren nach Anspruch 4, dadurch charakterisiert, daß das Lösungsmittel ausgewählt wird aus DMF, HMPT und DMSO.

6. Verfahren nach einem der Ansprüche 4 oder 5, dadurch charakterisiert, daß die Base ausgewählt wird aus tertiären Aminen und bevorzugt aus 4-Dimethylamino-pyridin, 1,4-Diaza-5-bicyclo[4.3.0]nonen, 1,4-Diaza-bicyclo[2.2.2]octan, 1,8-Diaza-7-bicyclo[5.4.0]undecen, Triethylbutylamin, Tri-n-butylamin und den alkalischen Carbonaten.

7. Verfahren nach einem der Ansprüche 1 bis 6, dadurch charakterisiert, daß das verwendete Phosphin Triphenylphosphin ist.

8. Verfahren nach einem der Ansprüche 1 bis 7, dadurch charakterisiert, daß der Azodicarbonsäurediester ein Alkylester oder Arylester ist.

9. Verfahren nach Anspruch 8, dadurch charakterisiert, daß der Diester bevorzugt Diisopropylazodicarboxylat (DIAD) oder Diethylazodicarboxylat (DEAD) ist.

10. Verfahren nach einem der Ansprüche 1 bis 9, dadurch charakterisiert, daß das Phosphin oder Phosphit und der Azodicarbonsäurediester im molaren Überschuß bezogen auf die Verbindung der Formel III verwendet werden.

11. Verfahren nach einem der Ansprüche 1 bis 10, dadurch charakterisiert, daß die Öffnungsreaktion in Gegenwart eines Überschusses Alkaliazid in einem polaren Lösungsmittel bewirkt wird.

12. Verfahren nach Anspruch 11, dadurch charakterisiert, daß dieser Überschuß in der Größenordnung von 1,5 bis 15 Equivalenten pro Mol der Verbindung der Formel IV liegt.

13. Verfahren nach einem der Ansprüche 11 oder 12, dadurch charakterisiert, daß das verwendete polare Lösungsmittel bevorzugt DMF ist.

14. Verfahren nach einem der Ansprüche 1 bis 13, dadurch charakterisiert, daß die Entschützung der Position 5' der Verbindung der Formel V mit Hilfe eines sauren Reagens von Typ Lewis-Säure oder protische Säure bewirkt wird.

15. Verfahren nach Anspruch 14, dadurch charakterisiert, daß die Entschützung in Gegenwart von Wasser mit einem Reagens durchgeführt wird, das ausgewählt wird aus Bortrifluoridetherat, Halogenwasserstoffsäuren und Essigsäure.
